Europäisches Patentamt

European Patent Office

Office européen des brevets

(19)

(11) Publication number: **0 117 087**

**B1**

(12) **EUROPEAN PATENT SPECIFICATION**

(45) Date of publication of patent specification: 16.06.87

(51) Int. Cl.⁴: **A 61 K 7/06**

(21) Application number: **84300630.5**

(22) Date of filing: **01.02.84**

(54) Hair tonic composition.

(30) Priority: **02.02.83 JP 14556/83**

(43) Date of publication of application:
**29.08.84 Bulletin 84/35**

(45) Publication of the grant of the patent:
**16.06.87 Bulletin 87/25**

(84) Designated Contracting States:
**AT BE CH DE FR GB IT LI LU NL SE**

(56) References cited:
**FR-A- 692 455**
**FR-E- 96 299**

**CHEMICAL ABSTRACTS, vol. 97, no. 25, 20th
December 1982, page 713, no. 214567s,
Columbus, Ohio, USA**

**CHEMICAL ABSTRACTS, vol. 91, no. 19, 5th
November 1979, page 346, no. 154361j,
Columbus, Ohio, USA, M.W. AKHTAR: "Lipase
induction in fungi"**

(73) Proprietor: **Suntory Limited
1-40 Dojimahama 2-chome Kita-ku
Osaka-shi Osaka-fu 530 (JP)**

(72) Inventor: **Yoshizumi, Hajime
6-1-612, Kosobe-cho 2-chome
Takatsuki-shi Osaka (JP)**
Inventor: **Amachi, Teruo
1-10, Hibarigaoka-Yamate 2-chome
Takarazuka-shi Hyogo-ken (JP)**
Inventor: **Kusumi, Takaaki
15-C-402, Yamate-cho 3-chome
Suita-shi Osaka (JP)**
Inventor: **Tanaka, Takaharu
5-1-801, Higashiawaji-cho 1-chome
Higashiyodogawa-ku Osaka-shi Osaka (JP)**
Inventor: **Ishigooka, Hiroshi
4-5, Nishichujo-cho
Ibaraki-shi Osaka (JP)**

(74) Representative: **Ford, Michael Frederick et al
MEWBURN ELLIS & CO. 2/3 Cursitor Street
London EC4A 1BQ (GB)**

EP 0 117 087 B1

**0 117 087**

## Description

The present invention relates to a novel hair tonic composition suitable for use in alleviating or preventing the generation of dandruff (or scurf) in hair and itching in the scalp and in accelerating the growth of hair. More specifically, it relates to a novel hair tonic composition containing, as an effective ingredient, products obtained by use of certain fungi.

The possession of a healthy and profuse head of hair throughout life is the ambition of most human beings. Various kinds of hair dressings, including hair tonic compositions, have been used for alleviating or curing epilation or depilation (i.e. the involuntary loss of hair and subsequent balding). However, although various kinds of disease appear, such as alopecia, the causes thereof and the mechanisms thereof are not fully understood. Accordingly, at present there are no truly effective agents for alleviating the epilation, accelerating the growth of hair, and further alleviating or curing the generation of dandruff in the hair and itching in the scalp.

FR—A—692455 mentions incubating *Aspergillus* or *Penicillium* in vegetable or animal oil, and states that the filtered product may be incorporated into a cosmetic preparation.

FR—E—96299 teaches the use of fungal cultures for preparing hair tonic compositions. The fungi are first cultured with bacteria and possible fungi include *Aspergillus* and *Penicillium*.

An object of the present invention is to provide a novel hair tonic composition capable of effectively alleviating the generation of dandruff and itching in the hair and scalp and also of exhibiting a remarkable and real acceleration of the growth of hair.

In accordance with the present invention, there is provided a hair tonic composition containing, as an effective ingredient, a product obtained from the interaction of at least one member selected from vegetable and animal fats and oils with at least one fungus, having a lipase producing capability, selected from fungi belonging to genera *Rhizopus, Penicillium, Geotrichum,* and *Sclerotium.*

The inventors have previously found that, after making a detailed comparative study of the microflora of the scalps of persons with healthy hair and those of persons with unhealthy hair (e.g., the generation of dandruff and itching in the scalp and abnormal depilation), *Staphylococcus capitis* constitutes all or most parts of the microflora in the scalp of those with healthy hair, and that, when the cells of *Staphylococcus capitis* or the treated products thereof are applied to the scalp, a remarkable growth effect of hair can be obtained. Furthermore, the inventors have clarified that the cells of *Staphylococcus capitis* have the lipase activity and testosterone 5α-reductase (i.e., "5α-reductase") inhibitory activity. The term "5α-reductase" denotes an enzyme which reduces testosterone to 5α-dihydrotestosterone. Based on these findings, the inventors have suggested that the above-mentioned activities are closely correlated with the growth effect of hair (see EP—A—102151).

The inventors have also found that, when the correlation of the above-mentioned lipase activity and 5α-reductase inhibitory activity with the hair growth effect is studied in detail, products containing fatty acids, as a main contituent, obtained from the interaction of lipase from *Staphylococcus capitis* with fats and oils, have a strong 5α-reductase inhibitory activity, and, furthermore, the above-mentioned fatty acids per se have an effective action on the growth of hair.

The inventors have further found that, after making a detailed inspection to find other microorganisms having properties similar to those of the above-mentioned *Staphylococcus capitis*, the products obtained from the interaction of vegetable and animal fats and oils with fungi belonging to genera *Rhizopus, Pencillium, Geotrichum,* and *Sclerotium* have an effective action on the growth of hair and also alleviate dandruff and itching.

The microorganisms or fungi used in the present invention are those having a lipase activity and belonging to genera *Rhizopus, Penicillium, Geotrichum,* and *Sclerotium,* and those capable of producing products having an effect on the hair growth when the fungi are interacted with fats and oils. These fungi can be any species and strains including those isolated from the natural occurring microorganisms and stored in any depository, those newly isolated, or artificial or natural mutants thereof, as long as they have the above-mentioned properties. Typical examples of these microorganisms are *Rhizopus delemar* ATCC-34612, *Penicillium cyclopium* ATCC 34613, *Geotrichum candidum* ATCC 34614, and *Sclerotinia libertiana* ATCC 22025 deposited in the American Type Culture Collection (ATCC). These microorganisms are freely available from ATCC.

If the microorganisms used in the present invention are desired to be freshly isolated, any conventional manner known in the art can be used. Furthermore, if the mutants thereof are desired to be obtained, any conventional mutation technique can be used in the present invention.

The fats and oils used in the present invention are those which can produce, upon interaction with the above-mentioned fungi, products having an effect on the hair growth. Examples of these fats and oils are vegetable oils such as olive oil, castor oil, cotton seed oil, coconut oil, soybean oil, palm oil, safflower oil, colza oil, rice bran oil, tsubaki oil (camellia oil), and sesame oil, and animal fats and oils such as tallow, lard, mutton tallow, mink oil, and whale oil. These fats and oils can be used alone or in any mixture thereof.

The methods for interacting the fats and oils with the above-mentioned fungi are not specifically limited. However, the following methods can be conveniently used from the practical point of view.

(1) The above-mentioned fungi are cultured in a culture medium containing the fats and oils;

2

(2) The above-mentioned fungi are cultured in a culture medium and the cultured cells of the fungi are then brought into contact with the fats and oils in an aqueous medium; or

(3) The above-mentioned methods (1) and (2) are combined.

When the above-mentioned method (1) is used, any culture conditions under which the fungi grows well can be used without limitation. Any nitrogen source which can be utilized by the fungi is used as a nitrogen source in the culture medium. Preferable examples of the nitrogen sources are organic nitrogen sources, for example, protein decomposition products such as casein peptones, soybean peptones, tripticase peptones, and casamino acids, and amino acids; extracts such as meat extracts and yeast extracts; soybean cake; and inorganic nitrogen sources such as ammonium salts and nitrates. These nitrogen sources can be used alone or in any mixture thereof.

Any carbon source which can be utilized by the fungi is used as a carbon source in the culture medium. Typical examples of such carbon sources are dextrin, galactose, glucose, fructose, mannose, sucrose, luctose, and glycerine. Furthermore, inorganic salts such as phosphates, hydrochlorides, magnesium salts, potassium salts, and sodium salts; vitamins; amino acids; or growth factors containing a large amount of these substances may be optionally used in the culture medium, depending upon the selection of the nitrogen sources.

In general, the above-mentioned nitrogen sources and carbon sources are independently used in a concentration of 0.1 g/liter to 100 g/liter, respectively, in the culture medium, depending upon the types of nitrogen and carbon sources. Furthermore, the above-mentioned inorganic salts and growth factors are generally used in a concentration of 0.01 g/liter to 50 g/liter, respectively, in the culture medium, depending upon the types of inorganic salts and the growth factors. The cultivation temperature is generally 20°C to 34°C, preferably 25°C to 28°C. The cultivation is generally effected at a pH of 3.5 to 8.5, preferably under aerobic conditions by, for example, a shaking culture or an aeration agitating culture. The cultivation time is generally 20 to 45 hours. The desired cultivation can be directly carried out. However, it is desirable that the precultured products obtained from a small scale preculture are inoculated into a culture medium.

The cultivation is effected by adding the above-mentioned fats and oils to the culture medium. The above-mentioned fats and oils can be contained in the culture medium prior to the cultivation or can be added to the culture medium during the cultivation after a certain amount of the cells is proliferated. The amount of the fats and oils is generally 1 g/liter to 250 g/liter, preferably 5 g/liter to 50 g/liter.

When the above-mentioned method (2) is used, the above-mentioned fungus is cultivated in the same manner as mentioned in method (1), except that no fats and oils are added to the culture medium. After the cultivation is completed, the cultured cells are brought into contact with the above-mentioned fats and oils. The contact of the cultured cells with the fats and oils can be most simply carried out, while appropriately stirring, after adding the fats and oils to the cultured medium containing the cultured cells of the fungus. However, if it is necessary to remove the medium components from the cultured fungus, the cells of the fungus are isolated from the cultured mixture by any conventional method, and the isolated cells are then suspended in an appropriate aqueous medium, for example, in a phosphate buffer solution, followed by the addition of the fats and oils. The pH and temperature conditions during the contact of the cells with the fats and oils are those within the ranges of the above-mentioned culture of the fungus.

The hair tonic composition according to the present invention contains, as an effective ingredient, the interaction products of the fungi with the fats and oils mentioned above. The interaction products can be incorporated into the hair tonic composition in various embodiments. Typical examples of such embodiments are as follows:

(a) The culture products obtained from the above-mentioned method (1), or the reaction mixtures obtained from the above-mentioned method (2) or (3);

(b) The liquid mixtures obtained by removing the cultured cells from the culture products of the above-mentioned method (1) or the reaction mixtures of the above-mentioned method (2) or (3);

(c) The mixtures containing fatty acids or the fatty acid mixtures separated from (and optionally purified to any extent) the culture products of the above-mentioned method (1), the reaction mixtures of the above-mentioned method (2) or (3), or the above-mentioned liquid mixtures (b).

In the practice of the above-mentioned embodiment (b), any separation or isolation technique conventionally used for separating or isolating cells from culture products or reaction products containing the cells can be used. Furthermore, in the practice of the above-mentioned embodiment (c), any conventional technique generally used in the separation and recovery of fatty acids contained in liquid media can be used. For example, the culture mixtures or reaction mixtures obtained from the interaction of the fungi with the fats and oils are directly used for recovering fatty acids therefrom. However, when the culture mixtures, which are obtained from the culture media containing as a main raw material soybean cake or other materials which remain as a solid substance after the cultivation, are used, the remaining solid contents are desirably removed from the cultured mixture together with a total or partial amount of the cultured cells. The recovery of the fatty acids from the above-mentioned cultured mixtures, reaction mixtures, or solid content removal mixtures obtained above can be advantageously effected by an extraction method from the commercial point of view. Any extraction solvent which can dissolve the desired fatty acids but is not substantially missible with water can be used. Examples of such extraction solvents are ethyl acetate, ether, benzene, chloroform, and hexane. These solvents can be used alone or in any mixture thereof during the extraction.

3

The extracts are then evaporized to remove the extraction solvents therefrom. The resultant residues can be used as an active ingredient in the hair tonic composition according to the present invention. However, when further purification of the active ingredients is desired, any conventional methods for purifying organic compounds having an acid group can be used. For example, the above-mentioned organic extracts are mixed with aqueous basic media such as basic buffers to form the salts of the desired active ingredients, whereby the active ingredients in the form of the salts are transferred into the aqueous phase in the dissolved form and, if desired, these active ingredients in the liquid media can be again extracted with the organic extraction solvent under an acidic condition. Alternatively, the crude products containing active ingredients are adsorbed by adsorbents such as silica gel and the adsorbed products can be separately eluted by solvents such as chloroform and benzene, alone or in any mixture.

As mentioned above, it is considered that there is a close correlation between the hair growth and the lipase activity and 5α-reductase inhibitory activity of the hair tonic composition applied to the surface of the scalp. The interaction products (i.e., the active ingredients) obtained from the interaction of the fungi with the vegetable and animal fats and oils have the 5α-reductase inhibitory activity, or both the 5α-reductase inhibitory activity and lipase activity. That is, active ingredients of the above-mentioned embodiments (a) and (b) have both the 5α-reductase inhibitory activity and lipase activity, whereas the active ingredients of the above-mentioned embodiment (c) do not have the lipase activity but have a strong 5α-reductase inhibitory activity due to the presence of concentrated fatty acids. These active ingredients of the above-mentioned embodiments (a), (b) and (c) can be most suitably used depending upon their characteristics in view of the properties of the base materials of the hair tonic compositions. ·

The amounts of the products (i.e., the active ingredients) obtained from the interaction of the fungi with the fats and oils largely depend upon the types of the active ingredients of the embodiments (a), (b), and (c), the types of the base materials of the hair tonic compositions, and the final forms of the hair tonic compositions. For example, the hair tonic composition of the present invention preferably includes 0.1% to 20% by weight of the active ingredients based on the weight of the hair tonic composition in the case of the above-mentioned embodiment (a) or (b). In the case of the active ingredients of the above-mentioned embodiment (c), the active ingredients are preferably included in an amount of 0.01% to 10% by weight based on the weight of the hair tonic composition and depending upon the purification degree of the fatty acids.

The hair tonic compositions of the present invention comprise the active ingredients of the above-mentioned embodiments (a), (b), or (c) contained in any conventional base materials of the hair tonic compositions. Examples of such base materials are as follows:

(I) Water or aqueous solutions;

(II) Aqueous alcoholic solutions mainly containing alcohols;

(III) Propylene glycol, liquid paraffin, and ceresin; and

(IV) Japan wax, hardened oils, lanolin derivatives, beeswax, and vaseline.

When the active ingredients of the above-mentioned embodiments (a) or (b) are incorporated into the hair tonic compositions to effectively utilize the lipase activity thereof, the above-mentioned base materials (I) are preferably used. On the other hand, when the 5α-reductase inhibitory activity is desired to be utilized, any base materials of cosmetic compositions can be used without limitation. For example, the above-mentioned base materials (I) to (IV) can be used alone or in any combination (e.g., solutions, emulsions).

In addition to the above-mentioned active ingredients, various conventional ingredients suitably used in the formulation of a hair tonic composition or a hair dressing composition can be incorporated in a conventional amount into the hair tonic composition of the present invention. Typical examples of such ingredients are cantharis tincture, Jaborandi tincture, Japanese green gentian (*Swertia Japonica*) extract, female hormones, vitamin E, nicotinic acid derivatives, other vitamins such as vitamin B groups, amino acids such as serine and methionine, acetyl choline derivatives, cepharanthine, photosensitizing dyes, menthol, salicylic acid, resorcinol, beeswax, cetanol, triethanol amine, borax, lower alcohol esters of $C_{14}$ to $C_{18}$ saturated fatty acids, cetanol amine, glycerol monostearate, glycerol, isopropyl myristate, castor oil, citric acid, plant gums, and perfumes. These ingredients can be optionally incorporated into the hair tonic composition of the present invention unless the desired effect of the present invention is impaired.

The final forms of the hair tonic composition according to the present invention can be any conventional form of hair tonic or hair dressing compositions, such as hair lotion, hair cream, hair liquid, hair oil, pomade, and hair stick. Other forms can also be utilized.

The hair growth effect of the active ingredients used in the present invention is not clearly understood but an attempted explanation is given hereinbelow without prejudice to the present invention.

Various theories have been proposed relating to the causes of depilation, epilation, dandruff, and itching. For example, an unbalanced hormone constitution theory, a nutrient relating theory, a seborrhea theory, and a genetic or hereditary theory are known; and it appears that there is a high correlation between the above-mentioned abnormal conditions and the development of sebaceous gland (see Masumi Inaba, "Mainichi Life" November, 1981, pages 26 to 35; "Saishin Keshohin Kagaku (Recent Cosmetics Science)", page 130 published by Yakuji Nippo Sha in 1980; Kenji Adachi et. al., "Biochemical and Biophysical Research Communication, *41* (4), pages 884 to 890 (1970); Susumu Takayasu et. al., Journal of Investigative Dermatology *74*, pages 187 to 191, 1980).

4

That is, when the sebaceous gland of the scalp is developed by nutrients, hormones or the like, the amount of 5α-reductase in the sebaceous gland increases. The level of 5α-dihydrotestosteron, more active androgen, becomes high. This tissue active androgen is transferred to hair papilla via blood vessels, thereby alleviating the activities of adenylcyclase in hair-matrix cells. As a result, it is believed that the size of hair-follicles is gradually reduced and, therefore, the hair becomes thin and downy, years of repetition of these processes cause baldness.

On the other hand, dandruff is formed because sebam is secreted and exudated to the surface of scalp in a large amount, due to the hypertrophy of sebaceous gland, and is mixed with horney or keratin peeled from the surface of the scalp. The dandruff thus formed inhibits dermal or skin respiration and the intake of nutrients into the hair-root portions. This also causes baldness.

Based on these mechanisms for generating depilation, epilation, and dandruff, the lipase activity and the 5α-reductase inhibitory activity are important and essential characteristics and properties which the hair tonic compositions should have. These activities also become a standard or criterion for scientifically evaluating the effect of the hair tonic or hair dressing compositions.

As mentioned above, the above-mentioned fungi used in the present invention have a lipase producing activity and a 5α-reductase inhibitory activity, and produce fatty acids having a strong 5α-reductase inhibitory activity when the lipase is allowed to interact with lipids or fats and oils. Accordingly, when the culture product or the reaction products of the above-mentioned embodiment (a) or the cell removed liquid thereof of the above-mentioned embodiment (b) are used as an active ingredient of the hair tonic composition according to the present invention, the hair tonic composition has a lipase activity and a strong 5α-reductase inhibitory activity. On the other hand, when the fatty acids recovered from the culture product, the reaction mixture, or the cell removed liquid thereof are used at an active ingredient, the resultant hair tonic composition has a strong 5α-reductase inhibitory activity.

According to the experiment system adopted by the inventors, the recovered products containing the fatty acids isolated from the culture products have a 50% inhibitory concentration ($IC_{50}$ value) of 0.20 mM against 5α-reductase. These fatty acids can inhibit 50% of the activity of the 5α-reductase in a concentration of 0.02% to 0.04%.

The hair tonic composition according to the present invention can accelerate the growth of hair and alleviate the generation of dandruff and itching in the scalp due to the fact that the active ingredients thereof have the above-mentioned 5α-reductase inhibitory activity or both the above-mentioned lipase activity and 5α-reductase inhibitory activity. Furthermore, the microflora of the scalp is maintained in or brought to a healthy state by the fatty acids contained in the hair tonic composition according to the present invention (these fatty acids are formed by the interaction of the above-mentioned fungi with the fats and oils) and/or the fatty acids formed by decomposing the lipids from the sebaceous gland with the lipase contained in the hair tonic composition according to the present invention. It is considered that when these actions or functions are combined or multiplied, they exhibit a strong hair growth acceleration effect.

The hair tonic composition according to the present invention has the 5α-reductase inhibitory activity or both the 5α-reductase inhibitory activity and the lipase activity as mentioned above, and when the hair tonic composition according to the present invention is applied to the human scalp or animal skins, strong hair growth acceleration effects can be provided. That is, when the hair tonic composition according to the present invention is applied to the human scalp, the depilation and epilation can be effectively alleviated, downy hairs become healthy, and the generation of dandruff and itching in the scalp can be prevented. Furthermore, when the hair tonic composition of the present invention is applied to animals, the growth velocity of the hair (fur) is remarkably increased.

The nonpathogenicity of the hair tonic composition according to the present invention has been confirmed. That is, the above-mentioned culture product and the purified fatty acids recovered therefrom were independently dispersed in an ethanol and the mixtures were spread on the skins of five rabbits once a day for 3 days in each dispersion. The ethanol was also spread in the same manner as a control. As a result, no abnormal condition was found in each case, as shown in Table 1.

TABLE 1

| Test dispersion | Irritation score |
| --- | --- |
| Control | 0 |
| 0.2% aqueous alcohol solution of the culture product | 0 |
| 0.02% aqueous alcohol solution of the fatty acid fractions | 0 |

The present invention also provides a method for applying, to hair, a hair tonic composition containing, as an effective ingredient, the product described hereinbefore or the analogous product derived from a fungus belonging to the genus Aspergillus. Thus a further aspect of the invention provides a method for applying, to hair, a hair tonic composition containing, as an effective ingredient, a product obtained from the interaction of at least one member selected from vegetable and animal fats and oils with at least one fungus, having a lipase producing capability, selected from fungi belonging to genera *Aspergillus,*

5

**0 117 087**

*Rhizopus, Penicillium, Geotrichum,* and *Sclerotium.* A still further aspect of the invention is a process for the cosmetic treatment of the human scalp which comprises applying thereto a composition containing a base material physiologically acceptable for exterior use and, as an effective ingredient, a product obtained from the interaction of at least one vegetable or animal fat or oil with at least one fungus, having a lipase producing capability, selected from fungi belonging to genera *Aspergillus, Rhizopus, Penicillium, Geotrichum,* and *Sclerotium.*

*Aspergillus niger* NRRL 337 is a typical example of Aspergillus, which has been deposited in the Agriculture Research Collection NRRL, and which is freely available from NRRL.

Examples

The present invention will now be further illustrated by, but is by no means limited to, the following examples, in which the preparation, application, and effect of the hair tonic composition of the present invention are specifically disclosed. Example 2 does not relate to a composition according to the invention, but to a composition which can be used in the method of applying, to hair, a hair tonic composition or in the process for the cosmetic treatment of the human scalp according to further aspects of the invention.

Example 1
Preparation of active ingredient

A 10 liter amount of a culture medium containing 100 g/liter of soybean peptone, 20 g/liter of glucose, 1 g/liter of sodium nitrate, 1 g/liter of $KH_2PO_4$, and 0.5 g/liter of $MgSO_4 \cdot 5H_2O$ was placed in a 20 liter jar fermentor and was then sterilized at a temperature of 120°C for 15 minutes. *Rhizopus delemar* ATCC 34612 cells were inoculated into the above culture medium ($1 \times 10^7$ spores/liter) and this was aerobically precultured at a temperature of 27°C for 4 days under agitation.

A 100 liter amount of the liquid culture medium having the same composition as mentioned above was charged into a fermentor (a charge capacity of 100 liters), and 5 liters of olive oil was then added to the liquid culture medium. After the liquid culture medium was sterilized at a temperature of 120°C for 15 minutes, 10 liters of the precultured product was added to the sterilized medium and was aerobically cultivated at a temperature of 27°C for 94 hours while stirring. A portion of the culture product was filtered by using Celite® as a filter aid, whereby hyphae were removed therefrom. Thus, the filtrate was obtained.

A 10 g amount of sodium chloride was dissolved in 100 ml of the filtrate obtained above and 100 ml of ethyl acetate was added thereto. The mixture was thoroughly stirred and the separated ethyl acetate layer was recovered. The ethyl acetate layer was concentrated to dryness. The resultant residue was dissolved in methanol and the methanol solution was again concentrated to dryness. The residue thus obtained was dissolved in benzene. The benzene solution was treated with silica gel column chromatography. The elution was carried out by using benzene, a mixture of benzene and chloroform (1:1 V/V), and chloroform, in that order. The chloroform eluate was concentrated to obtain the active ingredient containing fatty acids.

The lipase activity and 5α-reductase inhibitory activity of the above-mentioned culture products and the recovered and purified active ingredient were determined as follows:

Determination of lipase activity

A 4.5 ml amount of a Tris-hydrochloric acid buffer solution (pH=8.0), 1 ml of a 1/10 M calcium chloride solution, 1 g of a substrate (olive oil or rice bran oil), and 1 g or 1 ml of a sample were mixed together and the mixture was allowed to react at a temperature of 30°C for 1 hour while shaking. The amount of the formed fatty acids was titrated with a 1/20 M potassium hydroxide solution.

The results are as follows:

Lipase activity (culture product)

| Substrate | Activity |
|---|---|
| Olive oil | 105 unit* (μmol/min/ml) |
| Rice bran oil | 130 unit* (μmol/min/ml) |

\* The amount of the fatty acids, liberated from 1 ml of the culture solution per 1 minute, in terms of a titration amount of potassium hydroxide.

Determination of 5α-reductase inhibitory activity

Prostate gland of rats were homogenized and a specimen of testosterone 5α-reductase was then prepared by separating microsome from the homogenized liquid mixture. The conversion of the testosterone to 5α-dihydrotestosterone by the use of the above-prepared enzyme specimen was monitored by radioisotopically labelled testosterone. The reaction mixture was extracted with ethyl acetate and the extract was developed twice by silica gel thin layer chromatography (solvent system, dichloromethane: cyclohexane:acetone=15:4:1). The amount of 5α-dihydrotestosterone were determined from the intensities of the radioactivity.

6

Reaction

A 30 µl amount of a 0.05 M phosphate buffer (pH=6.6) containing 0.1% of BSA (bovine serum albumin) 10 µl of an enzyme specimen, 8.5 pmol of labelled testosterone, 50 nmol of a reduced form of NADP (nicotinamide adenine dinucleotide phosphate), and 10 µl of a test sample were mixed (final volume=50 µl). The mixture was incubated at a temperature of 25°C for 60 minutes.

The reaction was stopped by the addition of 50 µl of ethyl acetate, and the reaction mixture was extracted while vigorous shaking. The extract was developed in the same manner as mentioned above and the intensity of the radioisotope was measured by using a scintillation counter.

The above-mentioned determination was applied to the samples having various concentrations. The 5α-reductase inhibitory activity was obtained as an inhibition rate (%) or $IC_{50}$ (i.e., a 50% inhibition effective concentration).

The results are as follows:

5α-Reductase inhibitory activity

| Test sample | Radioactivity* | Inhibitory rate (%) |
|---|---|---|
| Culture product | 356 dpm | 97% |
| Control | 15651 dpm | — |

\* Since a portion of the formed 5α-dihydrotestosterone was further converted to adiols (3α- and 3β-17β-androstanediols) the radioactivity of the adiols was also counted.

As is clear from the results shown above, the culture products of the present invention have a strong 5α-reductase inhibitory activity. The 50% inhibitory concentration $IC_{50}$ of the fatty acid mixture obtained in Example 1 was 0.15 mM.

The fatty acid composition of the active ingredient in Example 1 determined by chromatography was as follows:

TABLE

| Fatty acid | Content (wt.%) |
|---|---|
| Palmitic acid | 1.1 |
| Palmitoleic acid | 0.3 |
| Stearic acid | 2.0 |
| Linolic acid | 18.8 |
| Linolenic acid | 0.8 |
| Oleic acid | 65.7 |

Of these fatty acids, palmitic acid, palmitoleic acid, oleic acid, and linoleic acid have an especially strong 5α-reductase inhibitory activity. Accordingly, fats and oils containing either one of these fatty acids can be used in the present invention.

Example 2

A 250 g amount of wheat bran and 200 g of water were mixed together and the mixture was placed in a dish type vessel. The mixture was then sterilized at a temperature of 120°C for 30 minutes to prepare a solid medium. Thus, twenty solid media were prepared. *Aspergillus niger* NRRL 337 cells were inoculated into these solid media and were then cultivated at a temperature of 27°C for 5 weeks.

After completing the cultivation, a 0.02 M acetic acid buffer solution (pH=5.6) at a temperature of 60°C was added to the culture mixture to extract the culture product. The lipase activity of the extract thus obtained was 80 unit (µmol/min/ml).

A 10 liter amount of the extract solution corresponding to 5 kg of wheat bran was allowed to react at a temperature of 25°C for 24 hours while stirring by adding 200 g of rice bran oil. The 5α-reductase inhibitory activity increased as follows:

| Reaction time | 5α-Reductase inhibitory activity* (unit) |
|---|---|
| 0 | 0 |
| 12 | 285 |
| 24 | 310 |

* The amount in which 50% of the 5α-reductase inhibitory activity under the experimental condition was inhibited is defined as 1 unit.

A 500 g amount of sodium chloride was dissolved in about 10 liters of the supernatant solution obtained above and the solution was extracted with 10 liters of chloroform. The chloroform extract solution was concentrated in vacuo to obtain an oily product.

The oily product thus obtained was extracted with hexane and the hexane extract was then adsorbed to an adsorption column containing 1 kg of silica gel packed therein. The elution was carried out by using benzene, a mixture of benzene and chloroform (1:1 V/V), chloroform, a mixture of chloroform and methanol (1:1 V/V), and methanol, in this order.

The 5α-reductase inhibitory activity of each eluted fraction were determined according to the above-mentioned method. The results are as follows:

TABLE

| Sample | Distribution (%) of 5α-reductase inhibitory activity |
|---|---|
| Reaction mixture | 100 |
| Eluate | |
| Benzene | 0 |
| Benzene-chloroform | 0 |
| Chloroform | 83 |
| Chloroform-methanol | 17 |
| Methanol | 0 |

Example 3

A 2 kg amount of soybean flour was added to 1 liter of water and was boiled. After sedimentation, the resultant supernatant solution was charged into a 20 liter jar fermentor. Furthermore, 300 g of corn steep liquor and 100 g of rapeseed oil were added and the pH was adjusted to 7.0. The mixture was sterilized at a temperature of 120°C for 30 minutes. *Pencillium cyclopium* ATCC 34613 cells were inoculated into the above culture medium and this was aerobically cultivated at a temperature of 27°C for 72 hours while agitating.

After completing the cultivation, the culture product was extracted in the same manner as in Example 1 to obtain the fatty acid containing product. The 5α-reductase inhibitory activity ($IC_{50}$) of the fatty acid containing product was 0.19 mM. The fatty acid composition of the fatty acid containing product was as follows:

| Fatty acid | Content (wt%) |
|---|---|
| Palmitic acid | 0.5 |
| Palmitoleic acid | trace |
| Stearic acid | 2.1 |
| Oleic acid | 20.5 |
| Linoleic acid | 15.1 |
| Linolenic acid | 5.6 |
| arachidic acid | trace |
| Eicosanoic acid | 0.3 |
| Elucic acid | 55.8 |

Example 4

A 40 g amount of rice bran, 30 g of corn steep liquor, and 2 g of ammonium phosphate were added to 10 liters of water and the mixture was charged into a 20 liter jar fermentor. The mixture was then sterilized at a temperature of 120°C for 30 minutes. A 20 g amount of sesame oil was added to the mixture.

8

# 0 117 087

*Geotrichum candidum* ATCC 34614 cells were inoculated into the resultant culture medium and this was aerobically cultivated at a temperature of 27°C for 70 hours.

The culture product was extracted in the same manner as in Example 1 to obtain 5α-reductase inhibitory substance, fatty acid containing product. The 5α-reductase inhibitory activity ($IC_{50}$) of the fatty acid containing product was 0.35 mM. The fatty acid composition of the fatty acid containing product was as follows:

| Fatty acid | Content (wt%) |
|---|---|
| Palmitic acid | 0.9 |
| Palmitoleic acid | trace |
| Stearic acid | 6.0 |
| Oleic acid | 54.1 |
| Linoleic acid | 38.8 |
| Linolenic acid | trace |

## Example 5

A 250 g amount of wheat bran and 200 g of water were mixed together and the mixture was placed in a dish type vessel. The mixture was then sterilized at a temperature of 120°C for 30 minutes to prepare a solid medium. Thus, twenty solid culture media were prepared. *Sclerotinia libertiana* ATCC 22025 cells were inoculated into these solid culture media and then solid cultured at a temperature of 27°C for 7 weeks.

After the cultivation, the culture mixture was extracted with water and 10 liters of the extract solution corresponding to 5 kg of wheat bran were obtained. A 500 g amount of lard was added to the extract solution and the mixture was allowed to react at a temperature of 30°C for 24 hours while stirring. The fatty acids were extracted from the reaction mixture and purified in the same manner as in Example 2.

The 5α-reductase inhibitory activity was increased as follows:

TABLE

| Reaction time (hr) | 5α-Reductase inhibitory activity (unit/ml) |
|---|---|
| 0 | 0 |
| 12 | 105 |
| 24 | 110 |

## Example 6 (Preparation form)

The following hair tonic compositions were formulated:

(1) Lotion type:

| Ingredient | Content (vol.%) |
|---|---|
| Tocopherol | 0.1 |
| Perfume | 1.0 |
| Preservative (5% sodium salicylate solution) | 1.0 |
| Culture product (Example 1) | 10.0 |
| Distilled water | to 100 |

(2) Hair cream type:

| Ingredient | Content (wt%) |
|---|---|
| Liquid paraffin | 50.0 |
| Polyethylene glycol | 1.0 |
| Tween 20 | 0.1 |
| Tocopherol | 0.1 |
| Perfume | 0.2 |
| Reaction mixture (Example 2) | 1.0 |
| Distilled water | to 100 |

9

(3) Aerosol type

| Ingredient | Content (wt%) |
|---|---|
| Polyoxyethylene *lanolin* | 1.0 |
| Lanolin alcohol | 2.5 |
| Glycerol fatty acid ester | 0.5 |
| Tocopherol | 0.1 |
| Perfume | 0.2 |
| Fatty acids (Example 1) | 0.62 |
| Denatured alcohol (absolute) | 25.08 |
| Propellant Freon 12/11 (40/60) | 70 |

(4) Hair Tonic type:

| Ingredient | Content (wt%) |
|---|---|
| Ethyl alcohol | 80.0 |
| Fatty acid (Example 3) | 0.2 |
| Distilled water | to 100 |

(5) Pomade

| Ingredient | Content (wt%) |
|---|---|
| Japan wax | 12.0 |
| Castor oil | 84.6 |
| Hardened oil | 2.0 |
| Tocopherol | 0.2 |
| Perfume | 0.2 |
| Fatty acids (Example 4) | 1.0 |

Example 7

Application of hair tonic composition

(1) Application of hair tonic type composition to the human scalp

The hair tonic type composition prepared in Example 6(4) was applied, twice a day, to the scalps of 10 men, each suffering from a large degree of itching, dandruff, and depilation at ages of 28 to 40; in an amount of 2 to 4 ml a day for 2 months.

The results are as follows:

TABLE

| Condition | Excellent | Effect Good | None |
|---|---|---|---|
| Dandruff | 7 | 2 | 1 |
| Itching | 10 | 0 | 0 |
| Depilation | 7 | 1 | 2 |

(2) Application of hair tonic type composition to rabbits

Ten week old male rabbits were shorn on the back. The hair tonic type composition prepared in Example 6(4) was applied, twice a day, to a half-side of the shorn portion of each rabbit for one week. As a control, the base material in Example 6(4) was also applied, twice a day, to the other half-side of the shorn portion of each rabbit for one week. The length of the grown fur was then measured.

The results are as follows:

10

# 0 117 087

TABLE

| Rabbit No. | Grown fur length | | Difference b |
| --- | --- | --- | --- |
| | Control side a | Test side a' | |
| 1 | 2.39±0.09 | 2.70±0.19 | 0.31 |
| 2 | 4.43±0.29 | 5.02±0.23 | 0.50 |
| 3 | 3.98±0.24 | 4.70±0.13 | 0.72 |
| 4 | 2.39±0.16 | 2.73±0.12 | 0.34 |
| 5 | 3.67±0.17 | 4.30±0.19 | 0.63 |
| 6 | 3.91±0.15 | 4.55±0.22 | 0.64 |
| 7 | 3.02±0.17 | 3.61±0.13 | 0.59 |
| 8 | 4.34±0.14 | 4.85±0.25 | 0.51 |
| 9 | 2.97±0.23 | 3.47±0.11 | 0.50 |
| 10 | 3.89±0.11 | 4.58±0.21 | 0.69 |
| Average | 3.499*[1] | 4.051*[2] | 0.552 |
| Standard error | — | — | 0.13 |

(Remarks)

a: Average fur growth length of control side (mm)±standard deviation

a': Average fur growth length of test side (mm)±standard deviation

b: Average fur growth length of test side—average fur growth length of control side (mm)

*[1]: Mean value of average growth length of control side

*[2]: Mean value of average growth length of test side

## Claims

1. A hair tonic composition containing, as an effective ingredient, a product obtained from the interaction of at least one member selected from vegetable and animal fats and oils with at least one fungus, having a lipase producing capability, selected from fungi belonging to genera *Rhizopus, Penicillium, Geotrichum*, and *Sclerotium*.

2. A hair tonic composition as claimed in Claim 1, wherein said product is obtained by cultivating the fungus in a culture medium containing a vegetable or animal fat or oil.

3. A hair tonic composition as claimed in claim 1, wherein said product is obtained by cultivating the fungus in a culture medium and then contacting the culture solution, an extract thereof, or the cultured cells with the vegetable or animal fat or oil in an aqueous medium.

4. A hair tonic composition as claimed in claim 1, wherein said product is the culture product or a cell free liquid of a reaction mixture obtained from the interaction of the vegetable or animal fat or oil with the fungus.

5. A hair tonic composition as claimed in claim 1, wherein said product is a fatty acid mixture isolated from the culture product, a reaction mixture obtained from the interaction of the vegetable or animal fat or oil with the fungus, or cell free liquid of the culture product or reaction mixture.

6. A hair tonic composition as claimed in any one of the preceding claims, wherein the vegetable and animal fat and oils are any of olive oil, castor oil, cotton seed oil, coconut oil, soybean oil, palm oil, safflower oil, colza oil, rice bran oil, tsubaki oil, sesame oil, tallow, lard, mutton tallow, mink oil, and whale oil.

7. A method for preparing a hair tonic composition according to any one of the preceding claims comprising incorporating product obtained from the interaction of at least one member selected from vegetable and animal fats and oils with at least one fungus, having a lipase producing capability, selected from fungi belonging to genera *Rhizopus, Penicillium, Geotrichum*, and *Sclerotium*, into the hair tonic composition.

11

# 0 117 087

8. A method for applying, to hair, a hair tonic composition containing, as an effective ingredient, a product obtained from the interaction of at least one member selected from vegetable and animal fats and oils with at least one fungus, having a lipase producing capability, selected from fungi belonging to genera *Aspergillus, Rhizopus, Penicillium, Geotrichum*, and *Sclerotium*.

9. A process for the cosmetic treatment of the human scalp which comprises applying thereto a composition containing a base material physiologically acceptable for exterior use and, as an effective ingredient, a product obtained from the interaction of at least one vegetable or animal fat or oil with at least one fungus, having a lipase producing capability, selected from fungi belonging to genera *Aspergillus, Rhizopus, Penicillium, Geotrichum*, and *Sclerotium*.

## Patentansprüche

1. Haartonikum, das als Wirkstoff ein Produkt enthält, welches durch Wechselwirkung mindestens eines aus den pflanzlichen und tierischen Fetten und Ölen ausgewählten Gliedes mit mindestens einem zur Bildung von Lipase befähigten Pilz erhalten ist, der der Gattung *Rhizopus, Penicillium, Geotrichum* oder *Sclerotium* angehört.

2. Haartonikum nach Anspruch 1, wobei dieses Produkt durch Züchten des Pilzes in einem ein pflanzliches oder tierisches Fett oder Öl enthaltenden Nährboden erhalten ist.

3. Haartonikum nach Anspruch 1, wobei dieses Produkt durch Züchten des Pilzes in einem Nährboden und anschließendes Zusammenbringen der Kulturlösung, eines Extraktes davon oder der gezüchteten Zellen mit dem pflanzlichen oder tierischen Fett oder Öl in einem wäßrigen Medium erhalten ist.

4. Haartonikum nach Anspruch 1, wobei dieses Produkt das Kulturprodukt oder eine zellfreie Flüssigkeit eines Reaktionsgemisches ist, das durch Wechselwirkung des pflanzlichen oder tierischen Fettes oder Öles mit dem Pilz erhalten ist.

5. Haartonikum nach Anspruch 1, wobei das Produkt ein aus dem Kulturprodukt isoliertes Fettsäuregemisch, ein durch Wechselwirkung des pflanzlichen oder tierischen Fettes oder Öles mit dem Pilz erhaltenes Reaktionsgemisch oder eine zellfreie Flüssigkeit des Kulturproduktes oder Reaktionsgemisches ist.

6. Haartonikum nach einem der vorhergehenden Ansprüche, wobei die pflanzlichen und tierischen Fette und Öle Olivenöl, Rizinusöl, Baumwollsaatöl, Kokosnußöl, Sojabohnenöl, Palmöl, Safloröl, Kolzaöl, Reiskleieöl, Tsubakiöl, Sesamöl, Talg, Schmalz, Hammeltalg, Nerzöl oder Walöl sind.

7. Verfahren zur Herstellung eines Haartonikums nach einem der vorhergehenden Ansprüche, bei dem ein Produkt, das durch Wechselwirkung mindestens eines Gliedes der pflanzlichen und tierischen Fette und Öle mit mindestens einem zur Bildung von Lipase befähigten Pilz erhalten wird, wobei der Pilz der Gattung *Rhizopus, Penicillium, Geotrichum* oder *Sclerotium* engehört, in das Haartonikumgemisch eingebracht wird.

8. Verfahren zum Auftragen eines Haartonikumgemisches, das als Wirkstoff ein Produkt enthält, welches durch Wechselwirkung mindestens eines Gliedes der pflanzlichen und tierischen Fette und Öle mit mindestens einem zur Bildung von Lipase befähigten Pilz, der der Gattung, *Aspergillus, Rhizopus, Penicillium, Geotrichum* oder *Sclerotium* angehört, erhalten ist, auf Haar.

9. Verfahren zur kosmetischen Behandlung der menschlichen Kopfhaut, bei dem auf diese ein Gemisch aufgetragen wird, das ein physiologisch verträgliches Grundlagenmaterial für die äußerliche Anwendung und einen Wirkstoff enthält, der ein Produkt ist, das durch Wechselwirkung mindestens eines pflanzlichen oder tierischen Fettes oder Öles mit einem zur Bildung von Lipase befähigten Pilz erhalten ist, der der Gattung *Aspergillus, Rhizopus, Penicillium, Geotrichum* oder *Sclerotium* angehört.

## Revendications

1. Une composition tonique pour les cheveux contenant, à titre d'ingrédient actif, un produit obtenu par l'interaction d'au moins un élément choisi parmi les matières grasses et huiles végétales et animales avec au moins un champignon présentant une aptitude à donner lieu à la formation de lipase, choisi parmi les champignons appartenant aux genres Rhizopus, Penicillium, Geotrichum et Sclerotium.

2. Une composition tonique pour les cheveux selon la revendication 1, dans laquelle ce produit est obtenu par culture du champignon dans un milieu de culture contenant une matière grasse ou huile végétale ou animale.

3. Une composition tonique pour les cheveux selon la revendication 1, dans laquelle ce produit est obtenu par culture du champignon dans un milieu de culture et mise ensuite en contact de la solution de culture, d'un extrait de celle-ci ou des cellules cultivées avec la matière grasse ou huile végétale ou animale dans un milieu aqueux.

4. Une composition tonique pour les cheveux selon la revendication 1, dans laquelle ce produit est le produit de culture d'un liquide exempt de cellules ou d'un mélange réactionnel obtenu par l'interaction de la matière grasse ou huile végétale ou animale avec le champignon.

5. Une composition tonique selon la revendication 1, dans laquelle ce produit est un mélange d'acide gras isolé dans le produit de culture, un mélange réactionnel obtenu par l'interaction de l'huile ou de la

**0 117 087**

matière grasse, végétale ou animale avec le champignon ou un liquide exempt de cellules du produit de culture ou du mélange réactionnel.

6. Une composition tonique pour les cheveux selon l'une quelconque des revendications précédentes dans laquelle la matière grasse et/ou les huiles animales et végétales représentent l'un quelconque parmi les produits suivants: huile d'olive, huile de ricin, huile de graine de coton, huile de noix de coco, huile de soja, huile de palme, huile de safran, huile de colza, huile de son ou de riz, huile de tsubaki, huile de sésame, suif, lard, suif de mouton, huile de vison, huile de baleine.

7. Une méthode de préparation d'une composition tonique pour les cheveux selon l'une quelconque des revendications précédentes qui consiste à incorporer le produit obtenu par l'interaction d'au moins un élément choisi parmi les matières grasses et huiles végétales et animales avec au moins un champignon présentant une aptitude à donner lieu à formation de lipase, choisi parmi les champignons appartenant au genre Rhizopus, Penicillium, Geotrichum et Sclerotium dans la composition tonique pour les cheveux.

8. Une méthode d'application aux cheveux d'une composition tonique pour les cheveux contenant, à titre d'ingrédient actif, un produit obtenu dans l'interaction d'au moins un élément choisi parmi les matières grasses et huiles végétales et animales avec au moins un champignon présentant une activité productrice de lipase choisi parmi les champignons appartenant au genre Aspergillus, Rhizopus, Penicillium, Geotrichum et Sclerotium.

9. Un procédé de traitement cosmétique du scalp humain qui consiste à y appliquer une composition contenant une matière de base physiologiquement acceptable à usage externe et, à titre d'ingrédient actif, un produit obtenu par l'interaction d'au moins une matière grasse ou huile végétale ou animale avec au moins un champignon présentant une aptitude à donner lieu à la formation de lipase, choisi parmi les champignons appartenant au genre Aspergillus, Rhizopus, Penicillium, Geotrichum et Sclerotium.

13